# EUROPEAN PATENT SPECIFICATION

(11) **EP 1 284 646 B1**
(45) Date of publication and mention of the grant of the patent: **22.02.2006**
(21) Application number: 01940462.3
(22) Date of filing: 11.05.2001
(51) Int. Cl.: A61B 5/0476

(54) **SYSTEM FOR DETECTING BRAIN ACTIVITY**
ANORDNUNG ZUR ERFASSUNG DER GEHIRNAKTIVITÄT
SYSTEME POUR OBSERVER L'ACTIVITE DU CERVEAU

(30) Priority: 16.05.2000 LU 90582
(43) Date of publication of application: 26.02.2003
(73) Proprietor: EUROPEAN COMMUNITY, 1049 Brussels (BE)
(72) Inventor: MILLAN, José, del R., I-21100 Varese (IT); TOPANI, Fabio, I-00144 Roma (IT); MOURINO, Josep, E-17007 Girona (ES)
(74) Representative: Beissel, Jean
(86) International application number: PCT/EP2001/005422
(87) International publication number: WO 2001/087153

(56) References cited:
- US-A- 4 013 068
- US-A- 4 846 190
- US-A- 5 038 782
- US-A- 5 222 503
- US-A- 5 279 305
- US-A- 5 730 146
- US-A- 6 011 991
- US-A- 6 052 619

## Description

### Introduction

The present invention generally relates to a brain interface system, and in particular to a system for the acquisition, processing, and classification of brain activity based on measurements of on-line EEG signals.

In the field of monitoring brain activity the use of electroencephalograph devices (EEG's) is well known. EEG's are used to measure and record small electrical signals, which occur on the surface of the scalp as a result of brain activity. Typically, an EEG system includes a plurality of electrodes attached at selected positions on the subject's scalp, a corresponding number of lead wires, and a processing console. Typically, each electrode is connected to the processing console via a separate lead wire. The processing console is provided for signal selection, amplification, and conditioning. Also included in EEG systems are means for measuring electrode impedance, calibrating equipment, and observing and permanently recording data processed by the processing console.

It is well known that the processing console and the observation and recording equipment are often incorporated within a single unit. The single unit, however, is too large to be easily transportable by the subject under observation. Further, because long wires between the electrodes and the processing console are impractical, the subject must remain relatively stationary when using most available EEG systems.

Some EEG systems have been developed to overcome the problem of the subject having to remain still during observation. These devices include portable recorders, which may be carried by the subject under observation. These systems do not, however, include means for contemporaneous observation of the record. It is well known that such observation is often desired.

One method for making EEG measurements and contemporaneous observations more practical is to replace the wire links between the electrodes and the processing console with wireless links. Thus, situations such as those described above, i.e. when long wires might encumber other simultaneous attention needed by the subject or when the mobility of the subject might be impaired would be at least partially resolved.

Newer studies indicate that electroencephalogram signals are a reliable mirror of mental activity and accordingly could be used as an alternative means of interaction with computers. The aim is to recognise from on-line EEG signals a few mental states and have them associated to simple commands for the user application to be controlled, such as "move wheelchair straight", "stop" and so on. A system for identifying brain activity, e.g. a brain interface of this kind, only requires users to be conscious of their thoughts and to concentrate sufficiently on those few mental tasks associated to the commands. Any other EEG pattern different from those corresponding to these mental tasks will be associated with the command "nothing", which has no effect on the computer-based system. By composing pattern sequences, users can operate computer-based systems, such as reading a web page, interacting with games, turning on appliances, or guiding a wheelchair. The immediate application is to extend the capabilities of physically-disabled people and let them access to the new set of services and opportunities deriving from the information society.

For these applications however, the interface should permit robust recognition of EEG patterns outside laboratory settings. This presumes the existence of an appropriate EEG equipment that should be compact, easy-to-use, and suitable for deployment in natural environments.

Unfortunately, the state of the art EEG systems are only suitable to operate under laboratory settings since the transmitted data are subject to interference and a specific mental state is very difficult to identify from these noisy signals. One of the major problems is that the signals produced at the different electrodes on the head are quite weak. This has made it difficult, and sometimes impossible, to obtain meaningful information from the signals at different electrodes even when such electrodes are attached to a person's head and signals are produced at such electrodes and are transmitted to a computer for subsequent analysis. Another reason is that the equipment associated with such electrodes is quite bulky and cumbersome and, even though bulky and cumbersome, is still unable sometimes to provide meaningful information.

US-A-4 013 068 discloses a system with the features of the first part of claim 1.

### Object of the invention

The object of the present invention is to provide a system for recognising mental states, which is better suited for the task of operating under real-life conditions.

### General description of the invention

In order to overcome the abovementioned problems, the present invention provides a system for identifying brain activity, as defined in the appended claims. The system comprises a plurality of electrodes, said electrodes being arranged on a supporting structure, e.g. a cap, to be placed on a skull of a person, said electrodes for producing signals in accordance with the brainwaves of said person, amplifying means for amplifying the electrical signals sensed by said electrodes, data processing means for processing said amplified signals, identifying a specific mental state from said signals and generating a signal indicative of said recognised mental state, and transmitter means for transmitting a signal corresponding to said identified mental state to a computer.

The electrodes are preferably arranged on the supporting structure in such a way that, when placed on the skull of a person, the electrodes are placed in predetermined anatomical positions on the head of the person. The electrodes are e.g. attached to the supporting structure so that their location is in accordance with the international 10-20 system.

The EEG data sensed by the electrodes is processed in the system itself and the mental state identified before transmission to a computer-controlled application. This means that only the detected state of mind has to be communicated to the computer, respectively the computer-controlled application. This of course minimises the influence of electromagnetic interference, which is a serious issue in prior art EEG systems.

Furthermore, all the electronic components of the system according to the invention can be implemented in an integrated circuit, which characterises by its small dimensions, its low weight and its low power consumption. Accordingly the elaboration unit of the proposed system stands out for its low dimensions and does not impede the freedom of movement of the user. Finally, due to the low power consumption, the system of the present invention can be battery powered, enabling a complete independence from line power and a good autonomy of the system. The powering by a battery pack bears the further advantage, that the system can be completely isolated from the 50/60 Hz interference of the line voltage.

All these factors make the system according to the present invention well suited for reliably operating in any environment.

In order to increase the comfort of the system, said amplifier means, said data processing means and said transmitter means can be incorporated in a wearable housing and preferably connected to said plurality of electrodes by means of a bi-directional power and data cable. The expression "wearable housing" means that said housing can be attached to the body of the user by means of a belt or the like. The housing further can contain the battery pack and comprise e.g. means for connecting a battery charger to said battery pack. The use of a bi-directionnal data and power reduces the number of individual connecting cables, thus reducing the overall weight of the system.

In a preferred embodiment, said data processing, means comprise filter means for filtering a signal pattern of a specific mental state from said amplified signals. Said filter means are preferably user-specific.

In an other embodiment, the system for identifying brain activity comprises storage means for storing reference electrode signal patterns of the mental states to be recognised, wherein said data processing means compares the pattern of said amplified signal to the pattern stored in said storage means. In this case said reference electrode signal patterns are preferably user specific reference patterns.

In the two embodiments, the filter means respectively the means for comparing the sensed pattern to the reference pattern form a robust neural classifier, which is implemented at the user-worn system for reliably recognise the desired mental states. The use of user-specific reference patterns respectively user specific filter means renders the system adaptive insofar that it can be optimised for the recognition of the effectively produced pattern of the user in question. The main reason preventing the development of universal brain interfaces is that not two people are the same, both physiologically and psychologically. This means that the interface should be adapted to its owner, thus allowing every single user to choose his/her most natural mental tasks to concentrate on (e.g., relaxation, visualisation, music composition, arithmetic, preparation of movements). In this way, users can regularly generate those individual EEG patterns that are better distinguished by their adaptive brain interface. This of course reduces the training time for the user and greatly enhances the recognition rate of the desired mental states.

In a very advantageous variant, the amplifying means comprises pre-amplifying means, which are arranged on said supporting structure in the vicinity of said electrodes, said pre-amplifier means pre-amplifying said electrical signals from said electrodes. Amplifying the signal directly on the electrodes minimises the effects of electromagnetic interference.

The pre-amplifying means preferably comprise one pre-amplifier for each electrode, each pre-amplifier being mounted on and associated with a respective electrode. Furthermore an analogue-to-digital converter (A/D converter) is advantageously associated with each pre-amplifier, for converting said amplified signal into a digital signal prior to communication of said signal to the data processing means. The A/D-converters can be connected to the data processing means by means of a bi-directional power and data cable.

In a preferred embodiment, said system further comprises receiver means for receiving data from a computer, said data comprising configuration settings for configuring adaptable parameters of said amplifying means and/or said data processing means and/or said transmitter means.

It has to be noted that said transmitter means comprises wireless transmitting means, e.g. infrared or radio frequency transmission means.

It will be appreciated that the present invention proposes a wearable system for complete analysis of brain activity. The system has a modular architecture and consists of a compact helmet with amplified EEG electrodes, on-board processing capabilities and flexible communication facilities. The hardware can easily be controlled by software that sets the suitable parameters. This allows the system to pre-process the data by hardware if necessary. Exploiting this option, some operations can be followed, e.g. removal of the 50/60 Hz electromagnetic interference, spatial filters, reference signal adaptation, monopolar and bipolar channel configuration etc. A suitable EEG-signal processing means for feature extraction and a robust neural classifier are implemented to reliably recognise the desired mental states.

The main advantages of the system according to the present invention include wearability, modularity and the analysis of the data at hardware level. Amplifying the signal directly on the electrodes minimises the effects of electromagnetic interference. This advantage, the small dimensions and the low weight allow the system to operate in any environment. An easy-to-use interface can be used to control the configuration of the system by software. The recognised mental states can be sent to the computer-controlled application through infrared or radiofrequency transmission (e.g. bluetooth protocol), or other wireless technologies to meet the specific needs.

### Detailed description with respect to the figures

The present invention will be more apparent from the following description of a not limiting embodiment with reference to the attached drawings, wherein
- Fig.1:: shows a schematic representation of an embodiment of a system for identifying brain activity;

- Fig.2:: shows a schematic representation of the electronic components of an embodiment of a system for identifying brain activity.

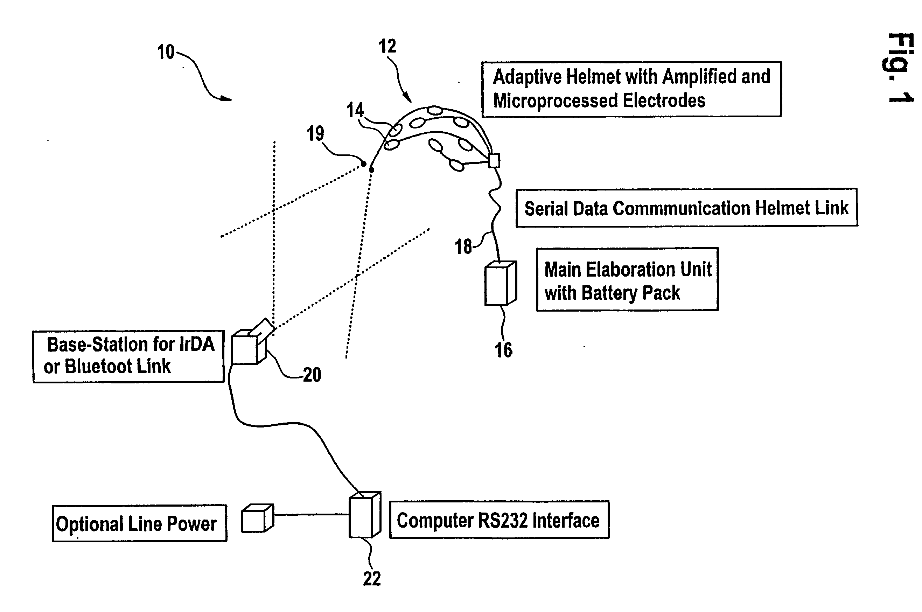

Fig. 1 shows a schematic representation of a wearable embodiment of a system 10 for identifying brain activity. The system 10 mainly comprises a helmet 12, i.e. a supporting structure like a cap, with a plurality of amplified electrodes 14. The amplified electrodes are e.g. attached to the supporting structure in accordance to the intemational 10-20 system and connected to a main elaboration unit 16 by means of a serial data communication link 18. The main elaboration unit includes the data processing means, at least a part of the transmitter means and a battery pack. An antenna 19 of the transmitter means can on the other hand be integrated into the helmet 12, the connection of the antenna 19 being realised by said serial data communication link 18.

The system 10 for identifying a brain activity communicates with a base station 20 connected to a user application 22, e.g. a computer, for controlling said user application by means of said identified mental state. The communication is implemented by a wireless link established between the system 10 and the base station 20.

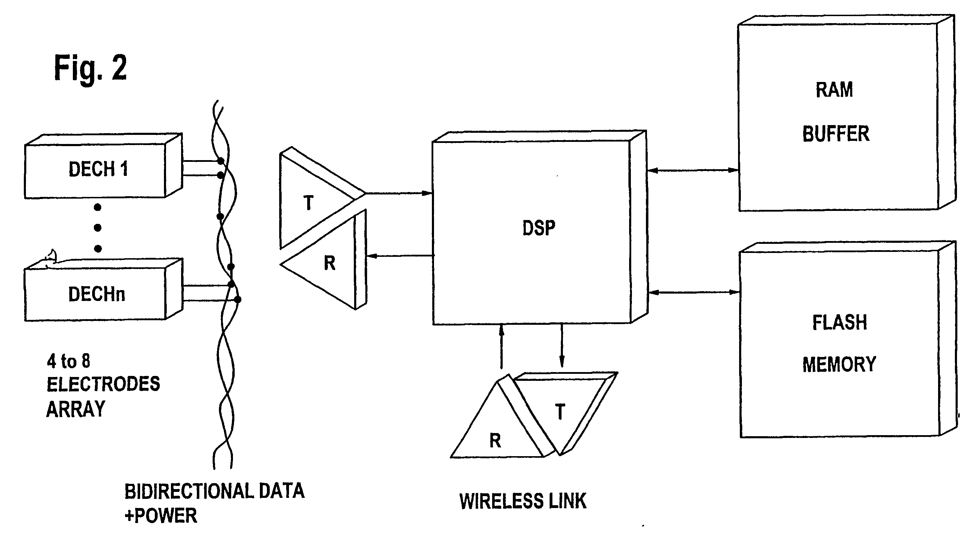

Fig. 2 shows a schematic representation of the electronic components of an embodiment of a system for identifying brain activity. These components mainly comprise an array of amplified and electrodes, which are connected via a communication link to a digital signal processor and its associated memory means. The signal processor processes the signals from said electrodes and extracts the mental state to be identified. The identified mental state is then communicated via a preferably wireless communication link to a base station connected to a user application to be controlled.

## Claims

1. Brain activity identification system (10) for controlling a computer-controlled application (12) comprising
a plurality of electrodes (14), said electrodes being arranged on a supporting structure (12) to be placed on a skull of a person, said electrodes for producing signals in accordance with the brainwaves of said person
amplifying means for amplifying the electrical signals sensed by said electrodes,
data processing means (16) for processing said amplified signals, identifying a specific mental state associated to a command of said computer-controlled application from said signals and generating a control signal for said computer-controlled application, said control signal being indicative of said recognised mental state, **characterised by**
wireless transmitter means (19,20) for wirelessly transmitting said control signal to said computer-controlled application.

2. System for identifying brain activity according to claim 1, comprising storage means for storing reference electrode signal patterns of the mental states to be recognised, wherein said data processing means compares the pattern of said amplified signal to the pattern stored in said storage means.

3. System for identifying brain activity according to claim 2, wherein said reference electrode signal patterns are user specific reference patterns.

4. System for identifying brain activity according to any one of claims 1 to 3, wherein said amplifying means comprise pre-amplifying means, said pre-amplifying means being arranged on said supporting structure in the vicinity of said electrodes, said pre-amplifier means amplifying said electrical signals from said electrodes.

5. System for identifying brain activity according to claim 4, wherein said pre-amplifying means comprise one pre-amplifier for each electrode, each pre-amplifier being mounted on and associated with a respective electrode.

6. System for identifying brain activity according to claim 5, wherein an analogue-to-digital converter is associated with each pre-amplifier for converting said amplified signal into a digital signal.

7. System for identifying brain activity according to claim 6, wherein said analogue-to-digital converters are connected to said data processing means by means of a bi-directional power and data cable.

8. System for identifying brain activity according to any one of claims 1 to 3, wherein said amplifier means, said data processing means and said transmitter means are incorporated in a wearable housing and connected to said plurality of electrodes by means of a bi-directional power and data cable.

9. System for identifying brain activity according to any one of the preceding claims, comprising receiver means for receiving data from a computer, said data comprising configuration settings for configuring adaptable parameters of said amplifying means and/or said data processing means and/or said transmitter means

10. System for identifying brain activity according to any one of the preceding claims, wherein said supporting structure is a cap

## Revendications

1. Système d'identification de l'activité cérébrale (10) destiné à contrôler une application contrôlée par ordinateur (12), comprenant
une pluralité d'électrodes (14), lesdites électrodes étant disposées sur une structure de soutien (12) à placer sur le crâne d'une personne, lesdites électrodes produisant des signaux en fonction des ondes cérébrales de ladite personne,
un moyen d'amplification pour amplifier les signaux électriques détectés par lesdites électrodes,
un moyen de traitement de données (16) pour traiter lesdits signaux amplifiés, identifier desdits signaux un état mental spécifique associé à une commande de ladite application contrôlée par ordinateur et générer un signal de contrôle pour ladite application contrôlée par ordinateur, ledit signal de contrôle étant révélateur dudit état mental identifié, **caractérisé par**
un moyen de transmission sans fil (19,20) pour transmettre sans fil ledit signal de contrôle à ladite application contrôlée par ordinateur.

2. Système pour identifier l'activité cérébrale selon la revendication 1, comprenant un moyen de stockage pour stocker des modèles de signaux d'électrodes de référence des états mentaux à reconnaître, dans lequel ledit moyen de traitement de données compare le modèle dudit signal amplifié au modèle stocké dans ledit moyen de stockage.

3. Système pour identifier l'activité cérébrale selon la revendication 2, dans lequel lesdits modèles de signaux d'électrodes de référence sont des modèles de référence spécifiques à l'utilisateur.

4. Système pour identifier l'activité cérébrale selon l'une quelconque des revendications 1 à 3, dans lequel ledit moyen d'amplification comprend un moyen de préamplification, ledit moyen de préamplification étant disposé sur ladite structure de soutien à proximité desdites électrodes, ledit moyen de préamplification amplifiant lesdits signaux électriques desdites électrodes.

5. Système pour identifier l'activité cérébrale selon la revendication 4, dans lequel ledit moyen de préamplification comprend un préamplificateur pour chaque électrode, chaque préamplificateur étant monté sur et associé à une électrode respective.

6. Système pour identifier l'activité cérébrale selon la revendication 5, dans lequel un convertisseur analogique-numérique est associé à chaque préamplificateur pour convertir ledit signal amplifié en un signal numérique.

7. Système pour identifier l'activité cérébrale selon la revendication 6, dans lequel lesdits convertisseurs analogiques-numériques sont connectés audit moyen de traitement de données à l'aide d'un câble d'alimentation et de données bidirectionnel.

8. Système pour identifier l'activité cérébrale selon l'une quelconque des revendications 1 à 3, dans lequel ledit moyen d'amplification, ledit moyen de traitement de données et ledit moyen de transmission sont incorporés dans un boîtier portable et sont raccordés à ladite pluralité d'électrodes à l'aide d'un câble d'alimentation et de données bidirectionnel.

9. Système pour identifier l'activité cérébrale selon l'une quelconque des revendications précédentes, comprenant un moyen de réception pour recevoir des données d'un ordinateur, lesdites données comprenant des réglages de configuration pour configurer des paramètres adaptables dudit moyen d'amplification et/ou dudit moyen de traitement de données et/ou dudit moyen de transmission.

10. Système pour identifier l'activité cérébrale selon l'une quelconque des revendications précédentes, dans lequel ladite structure de soutien est un casque.

## Patentansprüche

1. Anordnung zur Erkennung der Gehirntätigkeit (10) zur Steuerung einer rechnergesteuerten Anwendung (12), umfassend
eine Vielzahl von Elektroden (14), wobei die Elektroden auf einer auf den Schädel einer Person aufzusetzenden Trägerstruktur (12) angeordnet sind und geeignet sind, um Signale in Übereinstimmung mit den Gehimwellen der Person zu erzeugen,
Verstärkungsmittel zur Verstärkung der von den Elektroden erfassten elektrischen Signale,
Datenverarbeitungsmittel (16) zur Verarbeitung der verstärkten Signale, Erkennen eines, einem Befehl der rechnergesteuerten Anwendung zugeordneten spezifischen Geisteszustands aus den Signalen und Erzeugung eines Steuerungssignals für die rechnergesteuerte Anwendung, wobei das Steuerungssignal auf den erkannten Geisteszustand schliessen lässt, **gekennzeichnet durch**
drahtlose Sendemittel (19, 20) zum drahtlosen Senden des Steuerungssignals zur rechnergesteuerten Anwendung.

2. Anordnung zur Erkennung der Gehirntätigkeit nach Anspruch 1, die Speichermittel zum Speichern von Referenzelektrodensignalmustern der zu erkennenden Geisteszustände umfasst, wobei das Datenverarbeitungsmittel das Muster des verstärkten Signals mit dem im Speichermittel gespeicherten Muster vergleicht.

3. Anordnung zur Erkennung der Gehirntätigkeit nach Anspruch 2, wobei die Referenzelektrodensignalmuster nutzerspezifische Referenzmuster sind.

4. Anordnung zur Erkennung der Gehirntätigkeit nach irgendeinem der Ansprüche 1 bis 3, wobei die Verstärkungsmittel Vorverstärkungsmittel umfassen, die auf der Trägerstruktur in der Nähe der Elektroden angeordnet sind und die elektrischen Signale der Elektroden verstärken.

5. Anordnung zur Erkennung der Gehirntätigkeit nach Anspruch 4, wobei die Vorverstärkungsmittel einen Vorverstärker für jede Elektrode umfassen und jeder Vorverstärker auf einer jeweiligen Elektrode montiert ist und dieser zugeordnet ist.

6. Anordnung zur Erkennung der Gehirntätigkeit nach Anspruch 5, wobei ein Analog/Digitalwandler mit jedem Vorverstärker verbunden ist zur Umwandlung des verstärkten Signals in ein digitales Signal.

7. Anordnung zur Erkennung der Gehirntätigkeit nach Anspruch 6, wobei die Analog/Digitalwandler mit dem Datenverarbeitungsmittel durch ein Zweirichtungs-Strom- und Datenkabel verbunden sind.

8. Anordnung zur Erkennung der Gehirntätigkeit nach irgendeinem der Ansprüche 1 bis 3, wobei die Verstärkermittel, die Datenverarbeitungsmittel und die Sendemittel in ein tragbares Gehäuse eingebaut und mit der Vielzahl von Elektroden durch ein Zweirichtungs-Strom- und Datenkabel verbunden sind.

9. Anordnung zur Erkennung der Gehirntätigkeit nach irgendeinem der vorstehenden Ansprüche, die Empfangsmittel zum Empfang von Daten von einem Rechner umfasst, wobei die Daten Konfigurationseinstellungen zur Konfiguration adaptierbarer Parameter der Verstärkungsmittel und/oder Datenverarbeitungsmittel und/oder Sendemittel umfassen.

10. Anordnung zur Erkennung der Gehirntätigkeit nach irgendeinem der vorstehenden Ansprüche, wobei die Trägerstruktur eine Kappe ist.
